# EUROPÄISCHE PATENTSCHRIFT

(11) **EP 2 026 034 B1**
(45) Veröffentlichungstag und Bekanntmachung des Hinweises auf die Patenterteilung: **29.04.2020**
(21) Anmeldenummer: 08012024.9
(22) Anmeldetag: 03.07.2008
(51) Int. Cl.: G01B 11/25, A61C 13/00, A61B 1/05, A61B 1/00, A61B 1/247, A61B 5/06, A61B 5/107, A61B 5/00

(54) **Vorrichtung zur Ermittlung der 3D-Koordinaten eines Objekts, insbesondere eines Zahns**
Device for determining the 3D coordinates of an object, in particular a tooth
Dispositif destiné à la détermination de coordonnées 3D d'un objet, en particulier d'une dent

(30) Priorität: 16.08.2007 DE 102007038721; 14.12.2007 DE 102007060263
(43) Veröffentlichungstag der Anmeldung: 18.02.2009
(73) Patentinhaber: Carl Zeiss Optotechnik GmbH, 83115 Neubeuern (DE)
(72) Erfinder: Gandyra, Michael, Dipl.-Ing., 83022 Rosenheim (DE)
(74) Vertreter: Zinnecker, Armin

(56) Entgegenhaltungen:
- DE-A1- 19 526 526
- GB-A- 2 264 601
- US-A- 4 611 288
- US-A- 5 198 877
- US-A- 5 257 184
- US-A- 5 850 289

## Beschreibung

Die Erfindung betrifft einen Scanner zur Abtastung eines Objekts, insbesondere eines Zahns oder mehrerer Zähne oder eines Zahnmodells, und eine Vorrichtung zur Ermittlung der 3D-Koordinaten eines Objekts, insbesondere eines Zahns oder mehrerer Zähne oder eines Zahnmodells.

Vorrichtungen und Verfahren zur Ermittlung der 3D-Koordinaten eines Objekts sind bereits bekannt. In der EP 299 490 B2 wird ein Verfahren zur Herstellung von Zahnersatz beschrieben, bei dem Höhenschicht- oder Konturlinien auf den beschliffenen Zahn und seiner Umgebung erzeugt werden, die Linien mit einer optoelektronischen Einrichtung, insbesondere einer Videokamera, erfaßt werden, die erfaßten Werte in einen Rechner eingegeben werden und die räumliche Struktur des Zahnes und des Zahnersatzes berechnet wird. Anhand der so berechneten Struktur kann der Zahnersatz gefertigt werden.

Aus der US 4 611 288 A ist eine Vorrichtung zur optischen Abtastung eines Zahns bekannt.

Die US 5 257 184 A offenbart eine weitere Vorrichtung zur optischen Abtastung eines Zahns.

Aus der GB 2 264 601 A ist ein Scanner zur Abtastung eines Objekts, insbesondere eines Zahns oder mehrerer Zähne, nach dem Oberbegriff des Anspruchs 1 bekannt. Die US 5 198 877 A offenbart ein Tracking-System.

Die Erfassung der 3D-Koordinaten von Objekten, die sich in schwer zugänglichen Bereichen befinden, also insbesondere von Zähnen in der Mundhöhle eines Patienten, ist allerdings mit Schwierigkeiten verbunden.

Hiervon ausgehend liegt der Erfindung die Aufgabe zugrunde, einen verbesserten Scanner zur Abtastung eines Objekts, insbesondere eines Zahns, und eine verbesserte Vorrichtung zur Ermittlung der 3D-Koordinaten eines Objekts, insbesondere eines Zahns, vorzuschlagen.

Erfindungsgemäß wird diese Aufgabe durch einen Scanner mit den Merkmalen des Anspruchs 1, sowie durch einen Scanner mit den Merkmalen des Anspruchs 2 gelöst. Der Scanner dient zur Abtastung eines Objekts, insbesondere eines oder mehrerer Zähne, wobei einer oder mehrere oder alle Zähne präpariert sein können. Der Begriff des Präparierens umfaßt einerseits die zahnmedizinische Präparation, also beispielsweise das Beschleifen eines Zahnstumpfs, und andererseits die Präparation, die zum Scannen eines Zahns mit optischer Meßtechnik notwendig ist, beispielsweise das Einsprühen des zu scannenden Bereichs mit weißem Spray.

Der Scanner umfaßt einen Projektor zum Projizieren eines Musters auf das Objekt und eine Kamera, die eine Aufnahmeoptik und einen Bildsensor, insbesondere einen CCD-Sensor oder einen CMOS-Sensor, umfaßt. Erfindungsgemäß umfaßt die Aufnahmeoptik eine erste Bildoptik und eine zweite Bildoptik. Auf diese Weise wird von dem Scanner ein Stereobildpaar erzeugt. Die Bildoptiken können sich im Abstand voneinander befinden. Die optischen Achsen der Bildoptiken können in einem Winkel zueinander angeordnet sein. Sie sind vorzugsweise derart in einem Winkel zueinander angeordnet, daß sie auf einen übereinstimmenden Bereich des Objekts bzw. Zahns gerichtet sind. Im Ergebnis wird auf diese Weise der Bereich des Objekts bzw. Zahns mit zwei Kameras beobachtet.

Der erfindungsgemäße Scanner kann insbesondere als miniaturisierter Scanner ausgebildet werden. Er ist zum Abtasten von Zähnen im Mund eines Patienten besonders geeignet. Er ist allerdings auch für andere Anwendungen besonders geeignet, bei denen die abzutastenden Objekte schwer zugänglich sind. Insbesondere kann der erfindungsgemäße Scanner für das "Intra-Ear-Scanning", für eine endoskopische Digitalisierung und/oder in schwer zugänglichen Kavitäten und/oder Kanälen von Maschinen und/oder Apparaten verwendet werden.

Vorteilhafte Weiterbildungen sind in den abhängigen Ansprüchen beschrieben.

Nach der Erfindung umfaßt die Aufnahmeoptik einen Strahlteiler. Dem Strahlteiler können die Bilder von den Bildoptiken zugeführt werden. Der Strahlteiler kann diese Bilder auf den Bildsensor abbilden. Dabei können die Bilder aus den Bildoptiken auf jeweils unterschiedliche Bereiche des Bildsensors abgebildet werden. Die genaue Auslegung dieses spezifischen Strahlteilers ist in den Ansprüchen 1 und 2 definiert.

Es ist möglich, weitere Bildoptiken vorzusehen. Insbesondere können zwei weitere Bildoptiken vorgesehen werden, so daß insgesamt vier Bildoptiken vorhanden sind. Jedes Bild kann auf ein gesondertes Viertel des Bildsensors abgebildet werden.

Es ist ferner möglich, weitere Bildsensoren vorzusehen. Insbesondere kann für jede Bildoptik ein Bildsensor vorhanden sein.

Durch eine Aufnahme kann ein bestimmter Bereich des Objekts bzw. Zahns erfaßt werden. Um das gesamte Objekt bzw. den gesamten Zahn oder mehrere oder alle Zähne und möglicherweise auch deren Umgebung zu erfassen können mehrere Bilder sequentiell aufgenommen werden. Die Einzelaufnahmen können zu einer gesamten Objekt-Repräsentation zusammengefügt werden. Hierfür wird der Scanner um das Objekt bewegt, um mehrere oder alle Bereiche des Objektes zu erfassen. Da der Scanner jede Einzelaufnahme in seinem Koordinatensystem macht, ist es vorteilhaft, die Bewegung des Scanners zu erfassen, um die Einzelaufnahmen möglichst detailgetreu zusammenfügen zu können. Dieser Vorgang wird als "registrieren" bzw. "matchen" bezeichnet.

In Übereinstimmung mit Anspruch 2 weist der Scanner ein Trackingsystem auf, das wiederum einen oder mehrere Sensoren zur Erkennung von Lage und Orientierung des Scanners aufweist.

Vorzugsweise liefern der oder die Sensoren zur Erkennung von Lage und Orientierung des Scanners 6D-Informationen. Die 6D-Informationen bestehen aus drei Translationsinformationen und drei Rotationsinformationen. Hierdurch werden die Translation und die Rotation des Scanners vollständig erfaßt. Auf diese Weise wird für den Scanner ein Tracking-System gebildet.

In Übereinstimmung mit Anspruch 1 weist der Scanner einen oder mehrere Beschleunigungssensoren und/oder eines oder mehrere Gyrometer
auf. Durch die Beschleunigungssensoren kann die Scanner-Bewegung erfaßt und dadurch die Lage des Scanners bestimmt werden. Es ist möglich, durch die Beschleunigungssensoren die Lage des Scanners zu erkennen. Durch die Gyrometer können die Rotationen, also die Orientierung des Scanners erfaßt werden.

Die 6D-Information entspricht den sechs Freiheitsgraden, die definiert werden müssen, um einen Körper, nämlich den Scanner, im Raum hinsichtlich seiner Lage (Position) und Orientierung (Verdrehung) eindeutig zu definieren. Die 6D-Information des Scanners wird durch drei translatorische Komponenten und drei rotatorische Komponenten festgelegt.

Die zeitliche Integration eines Beschleunigungssensors liefert eine Geschwindigkeit. Deren weitere zeitliche Integration liefert eine Weg-Komponente. Hiervon ausgehend können drei Beschleunigungssensoren verwendet werden. Es sind allerdings auch Sensoren erhältlich, die alle drei translatorischen Komponenten gleichzeitig ermitteln.

Analoges gilt für Gyrometer, die Drehbeschleunigungen erfassen, deren zweimalige zeitliche Integration einen Drehwinkel liefert. Es ist möglich, drei Gyrometer zu verwenden, um die drei räumlichen Drehwinkel des Scanners zu erhalten. Es gibt allerdings auch Gyrometer, die alle drei räumlichen Drehwinkel liefern.

Ferner gibt es Sensoren, die alle drei Translationen und alle drei Rotationen liefern.

Stattdessen oder zusätzlich können an dem Scanner Marker für ein Tracking-System vorgesehen sein. Bei den Markern für das Tracking-System kann es sich um einen oder mehrere aktive Marker handeln. Insbesondere können Infrarot-Marker verwendet werden, die zeitlich sequentiell getriggert werden. Bei den Markern für das Tracking-System kann es sich allerdings auch um einen oder mehrere passive Marker handeln. Die passiven Marker können insbesondere codierte und/oder nicht codierte, reflektierende und/oder nicht reflektierende Marker sein. Die Marker können unterschiedliche Muster aufweisen.

Die an dem Scanner vorhandenen Marker werden zunächst vermessen. Damit ist die Position der Marker an dem Scanner bekannt. Die Marker werden dann von einer Tracking-Kamera verfolgt. Durch das Tracking-System können die Lage und die Drehstellung des Scanners bei den verschiedenen Aufnahmen erfaßt werden. Hierdurch werden Möglichkeiten verbessert oder geschaffen, die verschiedenen Einzelaufnahmen des Scanners zusammenzufügen.

Die der Erfindung zugrundeliegende Aufgabe wird bei einer Vorrichtung zur Ermittlung der 3D-Koordinaten eines Objekts, insbesondere eines Zahns, durch die Merkmale des Anspruchs 8 gelöst. Die Vorrichtung umfaßt einen erfindungsgemäßen Scanner zur Abtastung des Objekts bzw. Zahns und eine Auswerteeinrichtung zur Ermittlung der 3D-Koordinaten des Objekts aus den von dem Scanner aufgenommenen Bildern. Die Auswerteeinrichtung ist insbesondere ein Computer, insbesondere ein PC einschließlich der zugehörigen Software.

Die erfindungsgemäße Vorrichtung umfaßt ein Tracking-System zum Ermitteln der Lage und Orientierung des Scanners. Das Tracking-Systems kann dadurch gebildet werden, daß der Scanner einen oder mehrere Sensoren zur Erkennung von Lage und Orientierung des Scanners aufweist. Insbesondere kann das Tracking-System dadurch gebildet werden, daß der Scanner Beschleunigungssensoren und/oder Gyrometer aufweist. Es ist möglich, daß die Informationen des oder der Sensoren zur Erkennung von Lage und Orientierung des Scanners und/oder des oder der Beschleunigungssensoren und/oder des oder der Gyrometer, insbesondere deren 6D-Informationen, durch zeitliche Integration auf die Position und Drehstellung des Scanners zurückgerechnet werden. Stattdessen oder zusätzlich kann allerdings auch ein im Anspruch 2 definiertes Tracking-System mit am Scanner vorhandenen Markern verwendet werden, insbesondere ein Infrarot-Tracking-System.

Ausführungsbeispiele der Erfindung werden nachstehend anhand der beigefügten Zeichnung im einzelnen erläutert. In der Zeichnung zeigt
- Fig. 1: einen Scanner zur Abtastung einer Gruppe von Zähnen in einer schematischen Ansicht,
- Fig. 2: den Scanner gemäß Fig. 1, der zusätzlich noch Beschleunigungssensoren aufweist, und
- Fig. 3: den Scanner gemäß Fig. 1 mit Infrarot-Markern für ein Infrarot- Tracking-System.

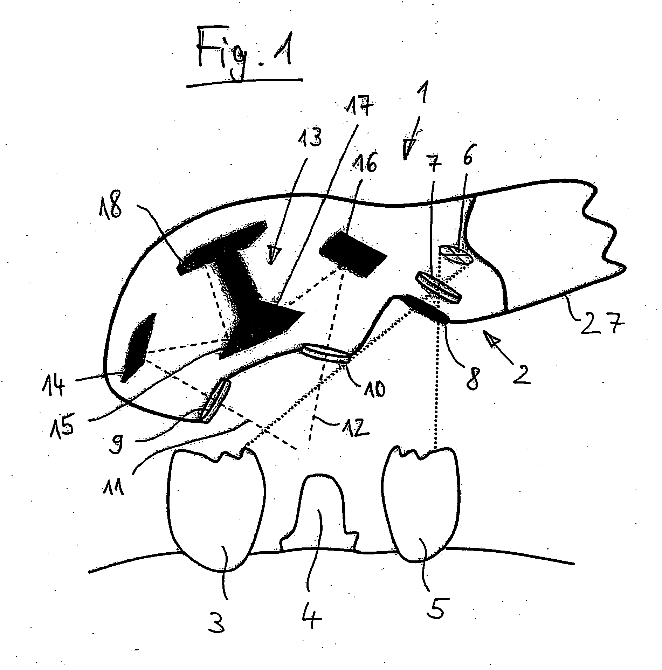

In Fig. 1 ist ein Scanner 1 dargestellt, der einen Projektor 2 zum Projizieren eines Musters auf die Zähne 3, 4, 5 aufweist. Der Zahn 4 ist beschliffen, die Nachbarzähne 3, 5 sind nicht beschliffen und bilden die Umgebung des beschliffenen Zahnes 4. Der Projektor 2 umfaßt eine Lichtquelle 6, ein Muster-Dia 7 und eine Projektionsoptik 8. Bei der Lichtquelle 6 kann es sich um eine Glühbirne oder um eine LED handeln. Statt des Muster-Dias 7 kann auch ein Durchlicht-LCD zum Einsatz kommen, die zur Bildung eines Musters angesteuert werden kann. Das Muster kann allerdings auch mittels DMD- oder LCOS-Verfahren projiziert werden.

In dem Scanner 1 ist ferner eine erste Bildoptik 9 und eine zweite Bildoptik 10 vorhanden, die voneinander beabstandet sind und deren optische Achsen 11, 12 einen Winkel zueinander bilden. Die Abstände der Bildoptiken 9, 10 und die Richtungen der optischen Achsen 11, 12 sind derart gewählt, daß sie auf einen gemeinsamen Bereich des beschliffenen Zahnes 4 gerichtet sind.

Der Scanner 1 umfaßt ferner einen Strahlteiler 13. Im optischen Weg von der Bildoptik 9 zu dem Strahlteiler 13 ist ein erster Spiegel 14 vorgesehen, der das Licht, das von dem beschliffenen Zahn 4 ausgeht und das durch die erste Bildoptik 9 abgebildet wird, auf den ersten Spiegel 15 des Strahlteilers 13 spiegelt. In entsprechender Weise ist ein zweiter Spiegel 16 im Strahlengang der zweiten Bildoptik 10 vorhanden, der das vom beschliffenen Zahn 4 ausgehende und von der zweiten Bildoptik 10 abgebildete Licht zur zweiten Spiegelfläche 17 des Strahlteilers 13 spiegelt. Von den Spiegelflächen 15, 17 des Strahlteilers 13 wird das Licht zu einem CCD-Sensor 18 gespiegelt. Das Bild aus der ersten Bildoptik 9 gelangt auf die linke Hälfte des CCD-Sensors 18, das Bild aus der zweiten Bildoptik 10 gelangt zur rechten Hälfte des CCD-Sensors 18. Die Bilder von den Bildoptiken 9, 10 werden dem Strahlteiler 13 zugeführt, der sie auf unterschiedliche Bereiche des CCD-Sensors 18 abbildet. Auf diese Weise wird auf dem CCD-Sensor 18 ein Stereobildpaar erzeugt, das von einer auf einem PC vorhandenen Software ausgewertet werden kann. Der CCD-Sensor 18 kann als geteilter CCD-Chip ausgebildet sein.

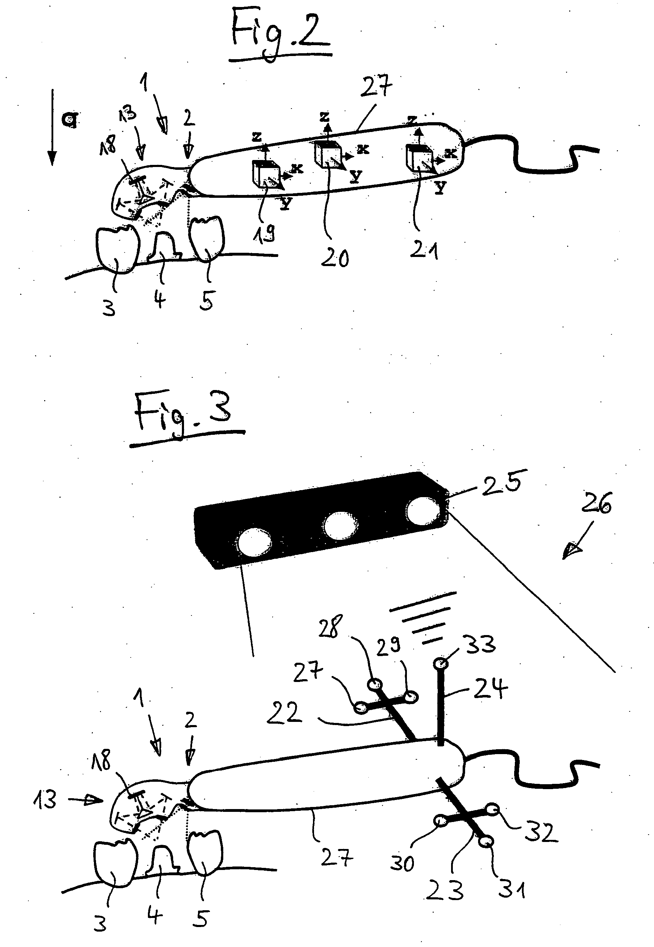

Der in Fig. 2 gezeigte Scanner entspricht dem in Fig. 1 gezeigten Scanner, so daß die bereits erläuterten Bestandteile nicht erneut beschrieben werden. Bei dem Scanner nach Fig. 2 sind zusätzlich noch Sensoren zur Erkennung von Lage und Orientierung des Scanners 3 vorhanden. Bei diesen Sensoren handelt es sich um drei Beschleunigungssensoren 19, 20, 21, die jeweils Informationen in x-, y- und z-Richtung liefern, aus denen 6D-Informationen abgeleitet werden können. Diese Beschleunigungsinformationen können zeitlich integriert werden, so daß daraus die räumliche Position und die Drehstellung des Scanners bestimmt werden können. Die Beschleunigungssensoren 19, 20, 21 befinden sich im Handgriff 27 des Scanners 1.

Fig. 3 zeigt den Scanner 1 nach Fig. 1 oder 2 mit Auslegern 22, 23, 24, an deren Enden Infrarot-Marker 27, 28, 29; 30, 31, 32; 33, insbesondere Infrarot-Dioden, vorgesehen sind, die Informationen für eine Infrarot-Tracking-Kamera 25 liefern. Die Infrarot-Marker 27 - 33 senden Infrarotstrahlen aus, die von der Infrarot-Tracking-Kamera empfangen werden. Das Tracking-System 26, das aus den Markern 27 - 33 und der Kamera 25 besteht, kann daraus die Lage und Orientierung des handgeführten Scanners 1 bestimmen.

Das Verfahren kann in der Weise durchgeführt werden, daß die Infrarot-Dioden 27 - 33 der Reihe nach aufleuchten. Dies wird durch die Tracking-Kamera 25 (bei der es sich um eine 3-Zeilen-Kamera handeln kann) zeitlich sequentiell erfaßt, woraus die Entfernung bestimmt werden kann. Der Verbund der Infrarot-Marker 27 - 33 ist durch seine Kalibrierung eindeutig bestimmt. Dadurch können Lage und Orientierung im Raum berechnet werden. Je mehr Marker ausgewertet werden können, um so genauer können Lage und Orientierung bestimmt werden (manche Marker werden beispielsweise während der Messung verdeckt.).

Durch die Erfindung kann ein meßtechnisches Verfahren durchgeführt werden, das auf der Auswertung von Stereobildpaaren basiert. Bei dem Verfahren wird ein Objekt mit zwei Kameras beobachtet. Mit geeigneten Algorithmen können in beiden Bildern dieselben Merkmale gefunden und korreliert werden. Bei kalibriertem System, wenn also die Lage und Orientierung beider Kameras bekannt sind, kann zu jedem Objektpunkt, der in beiden Kameras beobachtet werden kann, ein Abstandswert und damit eine 3D-Koordinate - im Koordinatensystem des Scanners - berechnet werden. Die Aufnahmeoptik wird mit einem Strahlteiler ausgestattet. Auf diese Weise kann der Scanner miniaturisiert werden. Das zu scannende Objekt, insbesondere ein zahnärztliche Präparation oder ein Objekt in einem schwer zugänglichen Bereich einer Maschine oder eines Apparates, kann aus zwei Richtungen beobachtet werden. Dabei wird die gleiche Szene aus zwei Richtungen auf nur einem Bildsensor bzw. CCD-Chip abgebildet. Eine Erweiterung auf mehr als zwei Richtungen, insbesondere auf vier Richtungen, ist möglich. Alle Bilder können auf einen CCD-Chip abgebildet werden. Es ist allerdings auch möglich, mehrere Bildsensoren zu verwenden.

Um in den verschiedenen Bildern dieselben Merkmale des Objekts zu finden, wird ein Muster auf das Objekt projiziert. Durch das projizierte Muster können in beiden Bildern die zugehörigen Bildbereiche gefunden werden.

Das zu projizierende Muster kann stochastisch oder geordnet sein. Es kann aus Linien oder Kreuzgittern bestehen. Es kann sich um ein zeitlich konstantes oder zeitlich veränderliches bzw. sequentielles Muster handeln. Das Muster kann ein beliebiges graphisches Muster sein (Punkte, Linien, Gitter etc.). Bei dem Muster kann es sich um ein Grauwertmuster oder um ein Farbmuster handeln. Das Muster kann mit Durchlicht projiziert werden, beispielsweise als Chrom-Maske (Dia) oder als LED-Projektion. Das Muster kann allerdings auch durch Reflektion projiziert werden, beispielsweise als LCOS- oder DLP-Projektion.

Je eine Bildaufnahme kann eine Datenaufnahme in Form einer 3D-Punktewolke erzeugen. Diese kann allerdings nur einen Teil der gesamten Repräsentation des realen Objektes darstellen. Aus diesem Grund können sukzessive mehrere Einzelaufnahmen von dem Objekt angefertigt werden, und diese Einzelaufnahmen können zu einer gesamten Objekt-Repräsentation zusammengefügt werden. Um dies zu erreichen kann der Scanner um das Objekt bewegt werden, um alle Bereiche des Objektes zu erfassen. Stattdessen oder zusätzlich kann der Scanner getrackt werden, z.B. durch ein außen angebrachtes Trackingsystem.

Durch die Erfindung wird ein Verfahren und eine Vorrichtung zur dynamischen Erfassung der Oberflächen von Objekten, insbesondere zur dynamischen intraoralen Erfassung der Oberflächen von zahnärztlichen Präparationen, geschaffen. Es wird ein Muster auf die Oberfläche projiziert, das zur Erzeugung der digitalen 3D-Daten aus zwei oder mehr Richtungen beobachtet und in entsprechend vielen 2D-Aufnahmen erfaßt wird. Es kann eine photogrammetrische Auswertung der 2D-Aufnahmen erfolgen, und die damit berechneten, zeitlich sequentiellen 3D-Einzelaufnahmen können durch die Ermittlung der Lage und Orientierung des Scanners im Raum mittels eines Trackingsystems zusammengesetzt werden.

Bei der photogrammetrischen Auswertung kann es sich um eine Auswertung von Stereobildpaaren handeln, die das zu vermessende Objekt aus zwei unterschiedlichen Richtungen darstellen. Es kann sich allerdings auch um eine Auswertung von mehreren Bildern aus mehreren Ansichten handeln, insbesondere um eine Auswertung von vier Bildern aus vier Ansichten. Bei dem Stereobildpaar kann es sich um ein Einzelbild handeln, das aus aus zwei Bildhälften besteht, die aus zwei Beobachtungsrichtungen aufgenommen wurden. Vier Einzelaufnahmen können in vier "Bildvierteln" zusammengefaßt werden.

Bei dem Trackingsystem kann es sich um ein optisches oder ein interferometrisches Trackingsystem handeln. Es ist allerdings auch möglich, das Trackingsystem durch an dem Scanner befestigte Sensoren zur Erkennung von Lage und Orientierung des Scanners und/oder Beschleunigungssensoren und/oder Gyrometer zu realisieren, mit deren Hilfe über die zeitliche Integration auf Geschwindigkeit und Position des Scanners zurückgerechnet werden kann. Die Beschleunigungssensoren können drei Translationsinformationen liefern. Die Gyrometer können drei Rotationsinformationen liefern, also Informationen über die Orientierung des Scanners. Besonders vorteilhaft ist es, sowohl Beschleunigungssensoren als auch Gyrometer vorzusehen, um dadurch 6D-Informationen zu erhalten.

Durch die Erfindung ist es möglich, die 3D-Koordinaten eines Objekts zu bestimmen. Dies kann dadurch geschehen, daß die 3D-Koordinaten über die Korrelation gleicher Merkmale in mehreren, insbesondere zwei, Bildern berechnet werden. Nach diesem Verfahren lassen sich in zwei Bildern eines Stereobildpaares, die aus unterschiedlicher Richtung aufgenommen worden sind, zugehörige gleiche Bildpunkte in beiden Bildern finden. Über die Kallibration der Bildoptiken und eine Triangulation lassen sich damit die 3D-Koordinaten der Objektpunkte berechnen. Um die Zuordnung der gleichen Merkmale in den Bildern eindeutig herstellen zu können, wird ein Muster projiziert. Das projizierte Muster dient diesem als Merkmalserkennung (feature recognition) bezeichneten Prozeß. Bei diesem Verfahren ist der Projektor nicht Bestandteil der Kalibirierung; er ist unabhängig von dem Stereo-Kamerasystem.

Es ist allerdings auch möglich, andere Verfahren zur Bestimmung der 3D-Koordinaten des Objekts durchzuführen. Insbesondere können Verfahren der "Einbild-Meßtechnik" durchgeführt werden, also Verfahren, mit denen man aus einer einzigen Bild-Aufnahme die 3D-Koordinaten des Objekts berechnen kann. Um dieses Verfahren durchführen zu können und um mehrere sequentielle Bildaufnahmen deselben Bereichs zu vermeiden, muß das aufgenommene Bild alle Informationen zur Berechnung der 3D-Koordinaten enthalten. Um dies zu gewährleisten wird üblicherweise ein Muster auf das Objekt projiziert. Um dieses Muster zu projizieren können unterschiedliche Eigenschaften des Lichts ausgenutzt werden. Beispielsweise kann es sich bei dem Muster um ein Kreuzgitter mit unterschiedlichen Farben handeln, wie dies in der DE 102 12 364 A1 beschrieben ist. Bei diesem Verfahren muß der Projektor zusammen mit der Kamera kalibriert sein. Das Verfahren kann in der Weise durchgeführt werden, daß mit dem Bild aus einer Bildoptik und mit dem projizierten Muster die 3D-Koordinaten berechnet werden und daß diese 3D-Koordinaten mit den Koordinaten, die aus einem Bild aus der anderen Bildoptik und aus dem projizierten Muster berechnet wurden, korreliert und optimiert werden. Die in diesem letzten Absatz aufgeführten Verfahren sind nicht Gegenstand der beigefügten Ansprüche.

## Patentansprüche

1. Scanner zur Abtastung eines Objekts (3, 4, 5), insbesondere eines Zahns oder mehrerer Zähne oder eines Zahnmodells, mit
einem Projektor (2) zum Projizieren eines Musters (7) auf das Objekt (3, 4, 5), und
einer Kamera, die eine Aufnahmeoptik mit einer ersten Bildoptik (9) und einer zweiten Bildoptik (10) und einen Bildsensor (18) umfaßt,
wobei
der Scanner (1) einen oder mehrere Beschleunigungssensoren (19, 20, 21) und/oder eines oder mehrere Gyrometer aufweist, und
die Aufnahmeoptik einen Strahlteiler (13) umfasst, der eine erste Spiegelfäche (15) und eine zweite Spiegelfläche (17) aufweist, wobei
die erste Spiegelfläche (15) dazu ausgelegt ist, von der ersten Bildoptik (9) abgebildetes und von einem ersten Spiegel (14) des Scanners auf die erste Spiegelfläche (15) gespiegeltes Licht auf einen ersten Bereich des Bildsensors (18) zu spiegeln, und die zweite Spiegelfläche (17) dazu ausgelegt ist, von der zweiten Bildoptik (10) abgebildetes und von einem zweiten Spiegel (16) des Scanners auf die zweite Spiegelfläche (17) gespiegeltes Licht auf einen zweiten zum ersten Bereich unterschiedlichen Bereich des Bildsensors (18) zu spiegeln.

2. Scanner zur Abtastung eines Objekts (3, 4, 5), insbesondere eines Zahns oder mehrerer Zähne oder eines Zahnmodells, mit
einem Projektor (2) zum Projizieren eines Musters (7) auf das Objekt (3, 4, 5),
einer Kamera, die eine Aufnahmeoptik mit einer ersten Bildoptik (9) und einer zweiten Bildoptik (10) und einen Bildsensor (18) umfasst, und
einem Tracking-System (26), das einen oder mehrere Sensoren zur Erkennung von Lage und Orientierung des Scanners aufweist,
wobei an dem Scanner (1) Marker (27, 28, 29; 30, 31, 32; 33) für das Tracking-System (26) vorhanden sind, und die Aufnahmeoptik einen Strahlteiler (13) umfasst, der eine erste Spiegelfäche (15) und eine zweite Spiegelfläche (17) aufweist, wobei
die erste Spiegelfläche (15) dazu ausgelegt ist, von der ersten Bildoptik (9) abgebildetes und von einem ersten Spiegel (14) des Scanners auf die erste Spiegelfläche (15) gespiegeltes Licht auf einen ersten Bereich des Bildsensors (18) zu spiegeln, und die zweite Spiegelfläche (17) dazu ausgelegt ist, von der zweiten Bildoptik (10) abgebildetes und von einem zweiten Spiegel (16) des Scanners auf die zweite Spiegelfläche (17) gespiegeltes Licht auf einen zweiten zum ersten Bereich unterschiedlichen Bereich des Bildsensors (18) zu spiegeln.

3. Scanner nach einem der vorhergehenden Ansprüche, mit weiteren Bildoptiken.

4. Scanner nach einem der vorhergehenden Ansprüche, mit weiteren Bildsensoren.

5. Scanner nach einem der Ansprüche 1 oder 2 bis 4, wobei der oder die Sensoren ausgelegt sind, 6D-Informationen zu liefern.

6. Scanner nach einem der Ansprüche 2 bis 4, wobei die Marker aktive Marker sind.

7. Scanner nach einem der Ansprüche 2 bis 4, wobei die Marker passive Marker sind.

8. Vorrichtung zur Ermittlung der 3D-Koordinaten eines Objekts (3, 4, 5), insbesondere eines Zahns oder mehrerer Zähne oder eines Zahnmodells, mit einem Scanner (1) nach einem der Ansprüche 1-7 und mit einer Auswerteeinrichtung zur Ermittlung der 3D-Koordinaten des Objekts (3, 4, 5) aus den von dem Scanner (1) aufgenommenen Bildern.

9. Vorrichtung nach Anspruch 8, mit einem Tracking-System (26) zum Ermitteln der Lage und Orientierung des Scanners (1).

## Claims

1. Scanner for scanning an object (3, 4, 5), in particular a tooth or a plurality of teeth or a tooth model, having
a projector (2) for projecting a pattern (7) onto the object (3, 4, 5), and
a camera, which comprises a recording optical unit having a first image optical unit (9) and a second image optical unit (10) and an image sensor (18),
wherein the scanner (1) has one or more acceleration sensors (19, 20, 21) and/or one or more gyrometers, and
the recording optical unit comprises a beamsplitter (13), which has a first mirror surface (15) and a second mirror surface (17), wherein
the first mirror surface (15) is designed to specularly reflect light, imaged by the first image optical unit (9) and specularly reflected onto the first mirror surface (15) by a first mirror (14) of the scanner, onto a first area of the image sensor (18), and
the second mirror surface (17) is designed to specularly reflect light, imaged by the second image optical unit (10) and specularly reflected onto the second mirror surface (17) by a second mirror (16) of the scanner, onto a second area of the image sensor (18), different to the first area.

2. Scanner for scanning an object (3, 4, 5), in particular a tooth or a plurality of teeth or a tooth model, having
a projector (2) for projecting a pattern (7) onto the object (3, 4, 5),
a camera, which comprises a recording optical unit having a first image optical unit (9) and a second image optical unit (10) and an image sensor (18), and
a tracking system (26), which has one or more sensors for recognizing the position and orientation of the scanner,
wherein markers (27, 28, 29; 30, 31, 32; 33) for the tracking system (26) are present at the scanner (1), and
the recording optical unit comprises a beamsplitter (13), which has a first mirror surface (15) and a second mirror surface (17), wherein
the first mirror surface (15) is designed to specularly reflect light, imaged by the first image optical unit (9) and specularly reflected onto the first mirror surface (15) by a first mirror (14) of the scanner, onto a first area of the image sensor (18), and
the second mirror surface (17) is designed to specularly reflect light, imaged by the second image optical unit (10) and specularly reflected onto the second mirror surface (17) by a second mirror (16) of the scanner, onto a second area of the image sensor (18), different to the first area.

3. Scanner according to any of the preceding claims, having further image optical units.

4. Scanner according to any of the preceding claims, having further image sensors.

5. Scanner according to any of Claims 1 or 2 to 4, wherein the sensor or sensors is/are designed to supply 6D information.

6. Scanner according to any of Claims 2 to 4, wherein the markers are active markers.

7. Scanner according to any of Claims 2 to 4, wherein the markers are passive markers.

8. Device for ascertaining the 3D coordinates of an object (3, 4, 5), in particular of a tooth or a plurality of teeth or a tooth model, having
a scanner (1) according to any of Claims 1-7 and having
an evaluation apparatus for ascertaining the 3D coordinates of the object (3, 4, 5) from the images recorded by the scanner (1).

9. Device according to Claim 8, having a tracking system (26) for ascertaining the position and orientation of the scanner (1).

## Revendications

1. Dispositif de balayage destiné à analyser un objet (3, 4, 5), notamment une dent ou plusieurs dents ou un modèle de dent, comprenant
un projecteur (2) destiné à projeter un motif (7) sur l'objet (3, 4, 5), et
une caméra, qui comporte une optique d'enregistrement pourvue d'une première optique d'image (9) et d'une deuxième optique d'image (10) et d'un capteur d'images (18),
le dispositif de balayage (1) possédant un ou plusieurs capteurs d'accélération (19, 20, 21) et/ou un ou plusieurs gyromètres, et
l'optique d'enregistrement comportant un diviseur de faisceau (13) qui possède une première surface réfléchissante (15) et une deuxième surface réfléchissante (17),
la première surface réfléchissante (15) étant conçue pour refléter sur une première zone du capteur d'images (18) la lumière représentée par la première optique d'image (9) et reflétée par un premier miroir (14) du dispositif de balayage sur la première surface réfléchissante (15), et
la deuxième surface réfléchissante (17) étant conçue pour refléter sur une deuxième zone du capteur d'images (18), différente de la première zone, la lumière représentée par la deuxième optique d'image (10) et reflétée par un deuxième miroir (16) du dispositif de balayage sur la deuxième surface réfléchissante (17).

2. Dispositif de balayage destiné à analyser un objet (3, 4, 5), notamment une dent ou plusieurs dents ou un modèle de dent, comprenant
un projecteur (2) destiné à projeter un motif (7) sur l'objet (3, 4, 5), et
une caméra, qui comporte une optique d'enregistrement pourvue d'une première optique d'image (9) et d'une deuxième optique d'image (10) et d'un capteur d'images (18), et
un système de poursuite (26) qui possède un ou plusieurs capteurs servant à reconnaître la position et l'orientation du dispositif de balayage,
des marqueurs (27, 28, 29 ; 30, 31, 32 ; 33) pour le système de poursuite (26) étant présents sur le dispositif de balayage (1), et
l'optique d'enregistrement comportant un diviseur de faisceau (13) qui possède une première surface réfléchissante (15) et une deuxième surface réfléchissante (17),
la première surface réfléchissante (15) étant conçue pour refléter sur une première zone du capteur d'images (18) la lumière représentée par la première optique d'image (9) et reflétée par un premier miroir (14) du dispositif de balayage sur la première surface réfléchissante (15), et
la deuxième surface réfléchissante (17) étant conçue pour refléter sur une deuxième zone du capteur d'images (18), différente de la première zone, la lumière représentée par la deuxième optique d'image (10) et reflétée par un deuxième miroir (16) du dispositif de balayage sur la deuxième surface réfléchissante (17).

3. Dispositif de balayage selon l'une des revendications précédentes, comprenant des optiques d'image supplémentaires.

4. Dispositif de balayage selon l'une des revendications précédentes, comprenant des capteurs d'images supplémentaires.

5. Dispositif de balayage selon l'une des revendications 1 ou 2 à 4, le ou les capteurs étant conçus pour délivrer des informations 6D.

6. Dispositif de balayage selon l'une des revendications 2 à 4, les marqueurs étant des marqueurs actifs.

7. Dispositif de balayage selon l'une des revendications 2 à 4, les marqueurs étant des marqueurs passifs.

8. Arrangement de détermination des coordonnées 3D d'un objet (3, 4, 5), notamment d'une dent ou de plusieurs dents ou d'un modèle de dent, comprenant un dispositif de balayage (1) selon l'une des revendications 1 à 7 et comprenant un appareil d'interprétation destiné à déterminer les coordonnées 3D de l'objet (3, 4, 5) à partir des images enregistrées par le dispositif de balayage (1).

9. Arrangement selon la revendication 8, comprenant un système de poursuite (26) destiné à déterminer la position et l'orientation du dispositif de balayage (1).
